# EUROPEAN PATENT SPECIFICATION

(11) **EP 2 976 023 B1**
(45) Date of publication and mention of the grant of the patent: **11.01.2017**
(21) Application number: 14729086.0
(22) Date of filing: 17.03.2014
(51) Int. Cl.: A61B 17/115

(54) **A CIRCULAR STAPLER HAVING A FOLDABLE ANVIL**
RUNDKLAMMERVORRICHTUNG MIT KLAPPBAREM AMBOSS
AGRAFEUSE CIRCULAIRE AYANT UNE ENCLUME PLIABLE

(30) Priority: 21.03.2013 TR 201303448
(43) Date of publication of application: 27.01.2016
(73) Proprietor: Sahin, Mustafa, Selcuklu, Konya (TR)
(72) Inventor: Sahin, Mustafa, Selcuklu, Konya (TR)
(74) Representative: Dereligil, Ersin
(86) International application number: PCT/TR2014/000076
(87) International publication number: WO 2014/149011

(56) References cited:
- US-A1- 2002 185 517
- US-A1- 2006 201 989
- US-A1- 2012 234 890
- US-A1- 2014 131 420

## Description

### Technical Field

The present invention relates to circular staplers used as automatic suture-positioner in the digestive system in surgical operations.

The present invention especially relates to a foldable anvil cap ensuring quick and secure anastomosis in providing the continuation of the circular stapler digestive system.

### Prior Art

Currently, the staplers are composed of two parts: a main body (handle) and an anvil attached to the end part of said body. Although said circular staplers which are available in the market make it possible to conduct quick and safe anastomosis, it is very limited for them to be used especially in laparoscopic surgery because of several shortcomings. The major shortcomings limiting the area of use in both open and laparoscopic surgery that:
1- A separate and large space is required to be formed on the bowel wall in order to locate the anvil into the bowel or stomach lumen that are going to be anastomosed,
2-It is difficult and time-wasting to close the open end with shirring suture even if it is located through the open end of the bowel and the stomach (for instance locating into esophagus following total gastrectomy) in both open and laparoscopic surgery.
3-In some cases (after esophagus surgery), the anvil is needed to reach the anastomosis line orally by being attached to the end of a catheter, as a result of which the microorganisms in the oral cavity and esophagus, spread into the abdomen, and thus the risk of infection increases.

Said applications cause difficulty of use and waste of time, and they limit the area of use. For example, it is not possible to use the available stapler anvils by attaching them onto the endoscope ends, and such an example of usage is not available.

In the research conducted in relation with the prior art, an application numbered CN201782789 has been found. In this application, a trigger mechanism created on a main body, back-pull handle, a modular anvil, and an anvil cap are disclosed. The invention discloses a foldable and movable anvil cap which can pass from the horizontal position to the parallel position.

US 2012/234890 A1 is considered to be the closest prior art of the present invention.

### The Objects of the Present Invention

In order to eliminate the disadvantages of the prior art, an object of the present invention is that the anvil cap can be folded onto itself by being divided into two and more parallel parts.

Another object of the present invention is that it can be made to go through a smaller space by reducing the surrounding area down to 1/2 and 1/3 of the diameter when it is open.

Another object of the present invention is that unlike the foldable anvil cap according to the invention numbered CN201782789, the anvil cap can be folded as multiple parts or foldable wings and it has a smaller diameter down to 1/2 and 1/3 when folded.

A further object of the present invention is that it can be produced in a detachable manner on the main body in a fixed or modular way (one-piece/modular), and go into the end that is going to be anastomosed through a small cut and conduct anastomosis by being opened in the shape of a shutter mechanism inside the lumen.

Another object of the present invention is that there is no need for a separate bigger cut to locate it inside the lumen.

A further object of the present invention is that it eliminates the need for locating the shirring suture in order to close the space that it goes into.

A further object of the present invention is that it provides advantage in both open and laparoscopic interventions.

Another object of the present invention is to expand the area of use in laparoscopic surgery.

A further object of the present invention is to eliminate the need for anastomoses conducted by linear staplers and by locating sutures manually in laparoscopic surgery, and the difficulty of conducting manual anastomosis.

A further object of the present invention is to expand the laparoscopic surgery indications thanks to the ease of use of the stapler.

Another object of the present invention is to make it possible to apply various surgical interventions through the natural openings (NOTES - Naturel Orifice Transluminal Endoscopic Surgery) of the body by being mounted to the end parts of endoscopy systems (Gastroscope, colonoscope, sigmoidoscope).

So as to fulfill the above-mentioned objectives, the present invention relates to a circular stapler according to the features of independent claim 1.

### Description of the Figures

Figure 1 is the general side view of the foldable anvil according to the present invention,
Figure 2 is the close-shot view of the detail a of the foldable anvil according to the present invention,
Figure 3 is the general top sectional view of the wings of the foldable anvil according to the present invention,
Figure 4 is the detail B view of the wings of the foldable anvil according to the present invention.
Figure 5 is the two-dimensional general side view of the foldable anvil according to the present invention when it is in a closed position.
Figure 6 is the general perspective view of the foldable anvil according to the present invention when it is in a closed position.

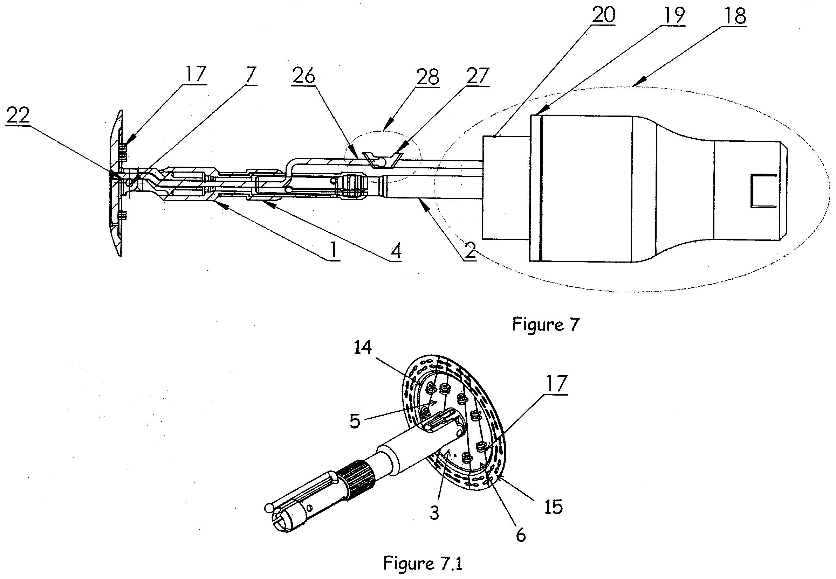

Figure 7 is the view of the stretching steel wire of the foldable anvil according to the present invention together with the connection clip.
Figure 7.1 is the perspective view of the wings of the anvil cap when they are opened together with the hinges providing their opening movement.

**Description of the Reference Numbers**

| Ref. No | Ref. Name |
|---|---|
| 1. | Carrier shaft body |
| 2. | Movable shaft |
| 2.1 | Wire space |
| 3. | Intermediate wing |
| 4. | Protective element |
| 5. | Right wing 1 |
| 6. | Left wing 1 |
| 7. | Connection point |
| 8. | Command wire |
| A, B, C | Wire ends |
| 9. | Punch surface |
| 10. | Withdrawing arm |
| 11. | Clamping arm |
| 12. | Pressure Control Section |
| 13. | Wire wrap pulley |
| 14. | Right wing 2 |
| 15. | Left wing 2 |
| 16. | Anvil cap |
| 17. | Hinge |
| 18. | Cartridge |
| 19 | Punch section |
| 20. | Blade mechanism |
| 21. | Foldable anvil |
| 22. | Wire crossing space |
| 23. | Connection end |
| 24. | Groove |
| 25 | Main body |
| 26 | Steel wire upper end connection points |
| 27 | Steel wire lower end connection points |
| 28 | Clips mechanism (with steel wire connection) |

### Detailed Description of the Invention

The present invention relates to a circular stapler having a foldable anvil (21) providing
secure anastomosis and comprising an anvil cap (16), a main body (25), a clamping arm (11) configured on said main body (25), a punch section (19) and a punch surface (9) corresponding to said punch section (19); characterized in comprising an anvil cap (16) comprising multiple foldable wings (3,5,6,14,15), a command wire (8) connected with said wings (3,5,6,14,15), a wire wrap pulley (13) directing said command wire (8). Said foldable anvil (21) comprises multiple hinges (17) connecting said wings (3,5,6,14,15) and providing the folding process, a connection point (7) connecting the intermediate wing (3) to the movable shaft (2) by means of a hinge (17), grooves (24) configured on each wing (3,5,6,14,15) for the connection of said command wire (8), connection ends (23) to which the command wire (8) that is embedded in said grooves (24), is fixed and a wire crossing space (22) configured at the center of said anvil cap (16). Said circular stapler comprises wire space (2.1) configured on said movable shaft (2).

The present invention is a foldable anvil (21) which makes it possible to conduct quick and secure anastomosis in providing the continuation of the digestive system of the circular staplers. In the art of foldable anvil (21), the anvil cap (16) according to the present invention is divided into two or more parts and these parts are connected to each other by means of hinges (17).

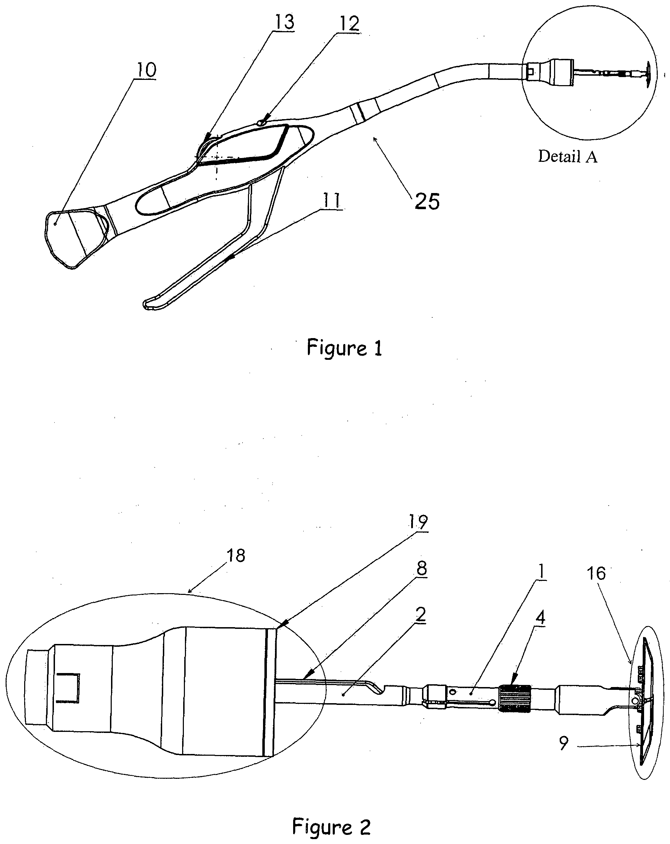

In Figure 1 and 2, the general side view of the foldable anvil according to the present invention is shown. According to the figure, there is a shaft withdrawing arm (10) enabling the anvil to be approached to the stapler carrier shaft body (25), combined with the cartridge (18) and locked. There is shown a clamping arm (11) enabling the textures to be punched after the staples inside the cartridge (18) go out, and providing the excess of texture to be cut by operating the blade mechanism (20).

As it is seen again in Figure 1, there is a pressure control section (12) so as to indicate the level of combination of the anvil according to the present invention with the cartridge (18). It comprises a pulley (13) around which the command wire (8) system that changes the position of the anvil cap (16) from parallel to vertical direction is collected and which opens the anvil wings (3,5,6,14,15) when they are in closed position.

In Figure 2, there is the detail A view of the foldable anvil according to the present invention. According to the figure, there is a carrier shaft body (1) carrying the anvil parts. The anvil according to the present invention has a movable shaft (2) enabling the stapler to be connected with the carrier shaft body (1) and providing the anvil to approach to the cartridge (18) part. The protective element (4) as a fixer is also shown in Figure 2. The protective element (4) is preferably made of a plastic-based substance.

A command wire (8) is used, which enables the anvil wings (3, 5, 6, 14, 15) to open and anvil cap (16) to change its position from parallel to vertical. The punch staples, on the other hand, are carried by the punch section (19).

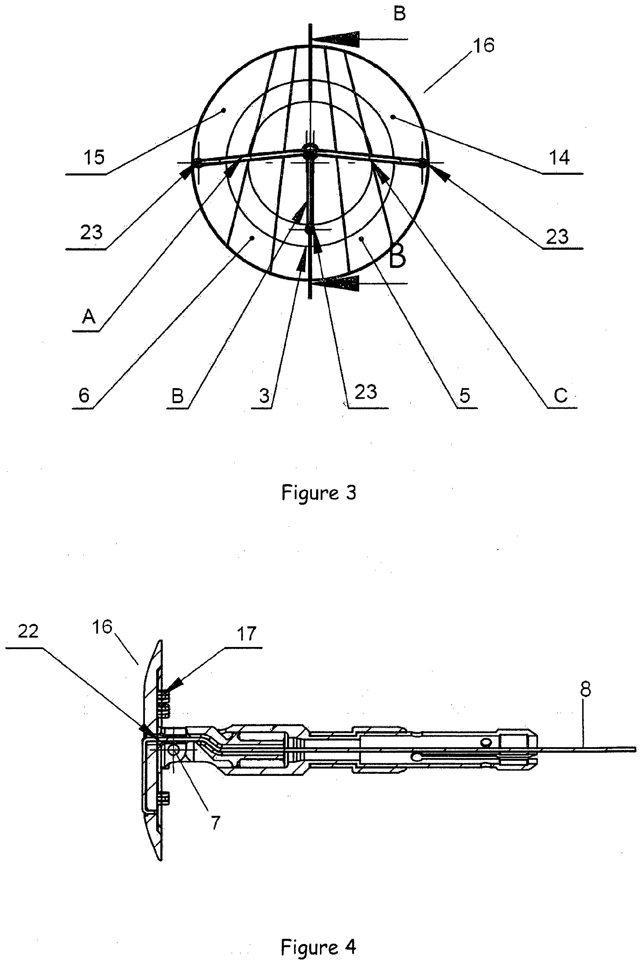

In Figure 3, the general front view of the wings (3, 5, 6, 14, 15) of the foldable anvil according to the present invention is shown. In the figure, an intermediate wing (3) providing the anvil cap (16) to be closed like a shutter and the parts to be attached to the carrier shaft body (1); a right wing 1 (5) and a left wing 1 (6) and right wing 2 (14) and left wing 2 (15), which ensure folding are shown.

In Figure 4, the detail B view of the wings (3, 5, 6, 14, 15) of the foldable anvil according to the present invention is shown. In this view, the connection point (7) is clearly shown.

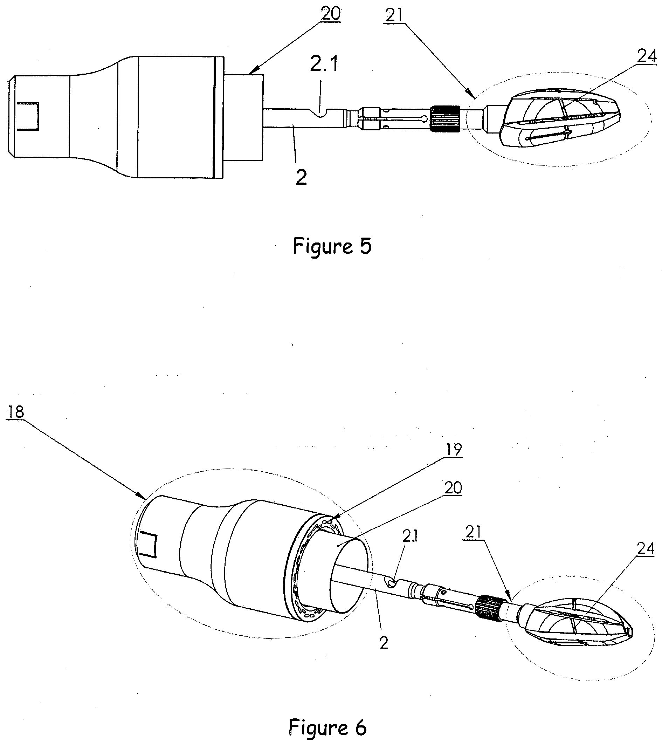

The divided anvil shown in Figure 1 is attached from the intermediate wing (3) connection point (7) to the movable shaft (2) by means of a hinge (17), and it is located onto the movable shaft (2) in a parallel way like a bayonet cap (see Figure 5). The parts (5, 6, 14, 15) located on both sides of the intermediate part (intermediate wing) (3) are wrapped around the movable shaft (2) like a shutter mechanism by being folded. The system to be configured in this way is made to proceed through a much narrower space than the width of the anvil when it is open, towards the bowel and stomach lumen.

After the anvil according to the present invention is made to proceed towards the lumen, a steel-based command wire (8) passing through the movable shaft (2) is used so as to open the closed shutter mechanism and to drop the anvil cap (16). After said command wire (8) according to the present invention is made to go through the wire crossing space (22) on the lower surface of the intermediate part (3), it is divided into three parts (A,B,C). One end of the wire (8) is attached to the connection end (23) corresponding to the carrier shaft body (1) of the intermediate main part (3) by passing through a groove (24) opened on the upper surface (See Figure 4). The other two ends of the wire (8) are made to proceed through a groove (24) similar to the side parts and the most outer wings (14, 15) are made to be attached from their middle parts. The foldable anvil (21) is in a folded position like a bayonet (see Figure 6).

The lower end connection points (27) on the carrier shaft body (1) are connected with the upper end connection points (26) on the movable shaft (2) by means of a clips mechanism (28).

A wire wrap pulley (13) to be configured on the middle part of said carrier shaft body (25), which can be hold manually, is combined with this steel command wire (8). Upon the pulley (13) is operated, the wire (8) is stretched. With the stretching process, the wire (8) will change the anvil from parallel position to a vertical position to the movable shaft (2), and then, as the stretching process continues, the side parts (5,6,14,15) are opened towards the outer part like a shutter mechanism. After the anvil cap (16) is opened entirely, the anvil is made to approach to the cartridge part (18) with the help of shaft withdrawing arm (10) on the main body (25), and the anastomosis process takes place after the clamping arm (11) is tightened.

In Figure 6, on the other hand, the general perspective view of the closed position of the foldable anvil (21) according to the present invention is shown. In this view, the anvil cap (16) of the foldable anvil (21) is folded like a bayonet by means of the wings (3,5,6,14,15), and a very small diameter is obtained. Therefore a much secure anastomosis can be performed with a much smaller diameter.

## Claims

1. A circular stapler having a main body (25), a clamping arm (11) configured on said main body (25), a punch section (19) and a foldable anvil (21) ensuring secure anastomosis and comprising an anvil cap (16) and a punch
surface (9) corresponding to said punch section (19), the anvil cap (16) comprising multiple foldable wings (3,5,6,14,15),
**characterised in that** said circular stapler further comprises
- a command wire (8) connected with said wings (3,5,6,14,15),
- a wire wrap pulley (13) directing said command wire (8).

2. A circular stapler according to Claim 1, **characterized in that** said
foldable anvil (21) comprises multiple hinges (17) connecting said wings (3,5,6,14,15) and providing the folding process.

3. A circular stapler according to Claim 1, **characterized in that** said multiple foldable wings comprise an intermediate wing (3) and **in that** said foldable
anvil (21) comprises a movable shaft (2) and a connection point (7) connecting the intermediate
wing (3) to the movable shaft (2) by means of a hinge (17).

4. A circular stapler according to any of the preceding claims, **characterized in that** said foldable anvil (21) comprises grooves (24) configured on each wing (3,5,6,14,15) for the connection of said command wire (8).

5. A circular stapler according to Claim 4, **characterized in that** said foldable
anvil (21) comprises connection ends (23) to which the command wire (8) that is embedded in said grooves (24), is fixed.

6. A circular stapler according to any of the preceding claims, **characterized in that** said circular stapler comprisies a wire space (2.1) configured on said movable shaft (2).

7. A circular stapler according to any of the preceding claims, **characterized in that** said circular stapler comprises a steel wire upper end connection point (26) controlling the movements of the anvil cap (16), steel wire lower end connection point (27) and a clips mechanism (28) combining these two ends.

8. A circular stapler according to any of the preceding claims, **characterized in that** said foldable anvil (21) comprises a wire crossing space (22) configured at the center of said anvil cap (16).

9. A circular stapler according to any of the preceding claims, **characterized in that** said foldable anvil (21) comprises a carrier shaft body (1) and a protective element (4) preferably made of plastic or a plastic-based substance configured on said carrier shaft body (1).

## Patentansprüche

1. Rundklammergerät, mit
einem Hauptkörper (25), einem Klammerarm (11),
der auf dem Hauptkörper (25) ausgestaltet ist, einem Stanzabschnitt (19) und einem faltbaren Amboss (21) zum Sicherstellen der sicheren Anastomose, und eine Ambosskappe (16), und
eine Stanzoberfläche (9) umfassend, die dem Stanzabschnitt (19) entspricht, wobei die Ambosskappe (16) zahlreiche faltbare Flügel (3, 5, 6, 14, 15) umfasst,
**dadurch gekennzeichnet, dass** das Rundklammergerät des Weiteren Folgendes umfasst
- einen Steuerungsdraht (8), der mit den Flügeln (3, 5, 6, 14, 15) verbunden ist,
- eine Drahtwicklungsrolle (13) zum Führen des Steuerungsdrahtes (8).

2. Rundklammergerät nach Anspruch 1, **dadurch gekennzeichnet, dass** der faltbare Amboss (21) zahlreiche Gelenke (17) umfasst, mit denen die Flügel (3, 5, 6, 14, 15) verbunden sind, und welche den Faltvorgang zur Verfügung stellen.

3. Rundklammergerät nach Anspruch 1, **dadurch gekennzeichnet, dass** die zahlreichen faltbaren Flügel einen Zwischenflügel (3) umfassen, und dass der faltbare Amboss (21) eine bewegliche Welle (2) und einen Verbindungspunkt (7) umfasst, mit welchem der Zwischenflügel (3) mit der beweglichen Welle (2) mittels eines Gelenks (17) verbunden ist.

4. Rundklammergerät nach einem der vorhergehenden Ansprüche, **dadurch gekennzeichnet, dass** der faltbare Amboss (21) Nuten (24) umfasst, die auf jedem Flügel (3, 5, 6, 14, 15) zur Verbindung mit dem Steuerungsdraht (8) ausgestaltet sind.

5. Rundklammergerät nach Anspruch 4, **dadurch gekennzeichnet, dass** der faltbare Amboss (21) Verbindungsenden (23) umfasst, an denen der Steuerungsdraht (8), der in den Nuten (24) eingebettet ist, befestigt ist.

6. Rundklammergerät nach einem der vorhergehenden Ansprüche, **dadurch gekennzeichnet, dass** das Rundklammergerät einen Drahtraum (2.1) umfasst, der auf der beweglichen Welle (2) ausgestaltet ist.

7. Rundklammergerät nach einem der vorhergehenden Ansprüche, **dadurch gekennzeichnet, dass** das Rundklammergerät einen oberen Endverbindungspunkt des Stahldrahtes (26), der die Bewegungen der Ambosskappe (16) steuert, einen unteren Endverbindungspunkt des Stahldrahtes (27) und einen Klipsmechanismus (28) umfasst, der diese beiden Enden miteinander kombiniert.

8. Rundklammergerät nach einem der vorhergehenden Ansprüche, **dadurch gekennzeichnet, dass** der faltbare Amboss (21) einen Drahtkreuzungsraum (22) umfasst, der in der Mitte der Ambosskappe (16) ausgestaltet ist.

9. Rundklammergerät nach einem der vorhergehenden Ansprüche, **dadurch gekennzeichnet, dass** der faltbare Amboss (21) einen Trägerwellenkörper (1) und ein Schutzelement(4) umfasst, das vorzugsweise aus Kunststoff oder einem auf Kunststoff basierten Material hergestellt ist, das auf dem Trägerwellenkörper (1) ausgestaltet ist.

## Revendications

1. Agrafeuse circulaire comprenant
un corps principal (25), un bras de serrage (11) configuré sur ledit corps principal (25), une section de perforation (19) et une enclume pliable (21) permettant de fixer une anastomose et comprenant un couvercle d'enclume (16) et
une surface de perforation (9) correspondant à ladite section de perforation (19), le couvercle d'enclume (16) comprenant une multitude d'ailettes pliables (3, 5, 6, 14, 15),
**caractérisée en ce que** ladite agrafeuse circulaire comprend en outre
- un fil de commande (8) relié auxdites ailettes (3, 5, 6, 14, 15),
- une poulie d'enroulement de fil (13) dirigeant ledit fil de commande (8).

2. Agrafeuse circulaire selon la revendication 1, **caractérisée en ce que** ladite enclume pliable (21) comprend une multitude de charnières (17) reliant lesdites ailettes (3, 5, 6, 14, 15) et assurant le pliage.

3. Agrafeuse circulaire selon la revendication 1, **caractérisée en ce que** ladite multitude d'ailettes comprend une ailette intermédiaire (3), et **en ce que** ladite enclume pliable (21) comprend un arbre mobile (2) et un point de connexion (7) reliant l'ailette intermédiaire (3) à l'arbre mobile (2) au moyen d'une charnière (17).

4. Agrafeuse circulaire selon l'une quelconque des revendications précédentes, **caractérisée en ce que** ladite enclume pliable (21) comprend des rainures (24) configurées sur chaque ailette (3, 5, 6, 14, 15) pour la connexion dudit fil de commande (8).

5. Agrafeuse circulaire selon la revendication 4, **caractérisée en ce que** ladite enclume pliable (21) comprend des extrémités de connexion (23) auxquelles le fil de commande (8) enrobé dans lesdites rainures (24) est fixé.

6. Agrafeuse circulaire selon l'une quelconque des revendications précédentes, **caractérisée en ce que** ladite agrafeuse circulaire comprend un espace à fil (2.1) configuré sur ledit arbre mobile (2).

7. Agrafeuse circulaire selon l'une quelconque des revendications précédentes, **caractérisée en ce que** ladite agrafeuse circulaire comprend un point de connexion d'extrémité supérieure de fil d'acier (26) commandant les déplacements du couvercle d'enclume (16), un point de connexion d'extrémité inférieure de fil d'acier (27) et un mécanisme de pince (28) combinant ces deux extrémités.

8. Agrafeuse circulaire selon l'une quelconque des revendications précédentes, **caractérisée en ce que** ladite enclume pliable (21) comprend un espace de croisement de fils (22) configuré au centre dudit couvercle d'enclume (16).

9. Agrafeuse circulaire selon l'une quelconque des revendications précédentes, **caractérisée en ce que** ladite enclume pliable (21) comprend un corps d'arbre de support (1) et un élément de protection (4) de préférence constitué de plastique ou d'une substance à base de plastique, configuré sur ledit corps d'arbre de support (1).
